# EUROPÄISCHE PATENTSCHRIFT

(11) **EP 2 648 667 B1**
(45) Veröffentlichungstag und Bekanntmachung des Hinweises auf die Patenterteilung: **07.12.2016**
(21) Anmeldenummer: 10794887.9
(22) Anmeldetag: 10.12.2010
(51) Int. Cl.: A61F 9/008

(54) **EINRICHTUNG ZUR SCHNEIDENDEN BEARBEITUNG DER KORNEA EINES HUMANEN AUGES MIT FOKUSSIERTER GEPULSTER LASERSTRAHLUNG**
DEVICE FOR CUTTING THE CORNEA OF A HUMAN EYE BY MEANS OF CUTS USING FOCUSED PULSED LASER RADIATION
DISPOSITIF POUR LE TRAITEMENT PAR INCISION DE LA CORNÉE D'UN OEIL HUMAIN PAR FAISCEAU LASER PULSÉ FOCALISÉ

(43) Veröffentlichungstag der Anmeldung: 16.10.2013
(73) Patentinhaber: WaveLight GmbH, 91058 Erlangen (DE)
(72) Erfinder: KRAUSE, Johannes, 90491 Nurnberg (DE); WÖLFEL, Mathias, 91052 Erlangen (DE); DONITZKY, Christof, 90542 Eckental/Eschenau (DE)
(74) Vertreter: Katérle, Axel
(86) Internationale Anmeldenummer: PCT/EP2010/007533
(87) Internationale Veröffentlichungsnummer: WO 2012/076033

(56) Entgegenhaltungen:
- WO-A1-2008/112292
- US-A1- 2008 058 777
- US-A1- 2008 058 841
- US-A1- 2008 114 386

## Beschreibung

Die Erfindung befasst sich mit der Erzeugung von Schnitten in der humanen Kornea mittels fokussierter gepulster Laserstrahlung. Insbesondere befasst sich die Erfindung mit der Präparation von Schnitten, welche einen Durchgang durch die Kornea eröffnen.

Für die Erzeugung von Schnitten mittels fokussierter Laserstrahlung in transparentem Material (transparent für die Laserstrahlung) wird als physikalischer Effekt der sogenannte laserinduzierte optische Durchbruch genutzt. Dieser führt zu einer lokalen Verdampfung des bestrahlten Materials, die man als Photodisruption bezeichnet. Die Photodisruption ist räumlich im wesentlichen auf das Gebiet des Fokus beschränkt. Durch Nebeneinandersetzen einer Vielzahl solcher Photodisruptionen können unterschiedlichste Schnittfiguren erzeugt werden.

Die photodisruptive Schnitterzeugung in der humanen Kornea mittels ultrakurzpulsiger fokussierter Laserstrahlung (mit Pulsdauern im Bereich von Femtosekunden) ist im Stand der Technik an sich bekannt. Beispielsweise ist diese Technik der Schnitterzeugung vielfach für die Präparation des Flaps bei einer LASIK-Operation (LASIK: Laser in-situ Keratomileusis) vorgeschlagen worden.

Korneale Schnitte sind jedoch nicht nur bei LASIK-Operationen vonnöten sondern bei einer ganzen Reihe anderer Operationsformen. Als ein diesbezügliches Beispiel kann die intrakorneale Lentikelextraktion genannt werden, bei der durch zwei übereinanderliegende, einander an den Rändern berührende Flächenschnitte ein linsenförmiges Gewebestück in der Kornea herausgetrennt wird.

Im Rahmen der Erfindung geht es hingegen um schlitzförmige Schnitte, die von der Vorderfläche der Kornea zur Rückfläche durchgehen. Solche Schnitte können einen Zugangskanal zu den innenliegenden Bereichen des Auges eröffnen und werden beispielsweise bei Operationen der humanen Linse oder bei lamellären Keratoplastiken des kornealen Endothels benötigt, um medizinische Instrumente oder/und medizinisches Material (z.B. künstliche Ersatzlinse, Spendergewebe, etc.) einführen und entnehmen zu können (wie beispielsweise in WO 2008/112292).

Aufgabe der Erfindung ist es, eine minimalinvasive Alternative zur mechanischen Erzeugung durchgehender Schnitte in der Kornea mittels eines Skalpells aufzuzeigen.

Zur Lösung dieser Aufgabe sieht die Erfindung eine Einrichtung zur Erzeugung mindestens eines von der Rückfläche bis zur Vorderfläche der Kornea eines humanen Auges durchgehenden schlitzförmigen Schnitts vor, wobei die Einrichtung eine Lasereinrichtung zur Erzeugung wenigstens eines Teils des Schnitts mit fokussierter gepulster Laserstrahlung umfasst, wobei die Lasereinrichtung steuerbare Komponenten zur Ortseinstellung des Strahlungsfokus, einen Steuerrechner zur Steuerung dieser Komponenten und ein Steuerprogramm für den Steuerrechner umfasst, wobei das Steuerprogramm Instruktionen enthält, welche dazu ausgelegt sind, bei Ausführung durch den Steuerrechner die Erzeugung zumindest eines von der Rückfläche der Kornea ausgehenden Teils des Schnitts zu bewirken, wobei zumindest dieser Teil des Schnitts einen Querschnittsverlauf - bei Betrachtung in Richtung von der Vorderfläche zur Rückfläche - aufweist, welcher von einer zur Augenoberfiläche senkrechten Geraden abweicht. Die verwendete Laserstrahlung hat vorzugsweise Pulsdauern im Bereich von weniger als 1 Pikosekunde. Ihre Wellenlänge kann beispielsweise im niederen infraroten oder im ultravioletten Bereich liegen, solange sie eine ausreichende Transmission in die Tiefe der Kornea gestattet.

Unter einem schlitzförmigen Schnitt wird hier ein zweidimensional ausgedehnter Schnitt verstanden, dessen Schnittdicke klein, insbesondere verschwindend klein gegenüber der Flächenausdehnung ist. Beispielsweise kann die Schnittdicke nur einer einzigen Photodisruption entsprechen, d. h. die Photodisruptionen werden nur in einer einzigen Ebene aneinandergereiht. Der im Rahmen der Erfindung betrachtete Querschnittsverlauf von Vorder- zu Rückfläche der Kornea ist als Verlauf in einem Längsquerschnitt zu verstehen. Die Länge des Schnitts ist durch den Abstand von Vorderfläche zu Rückfläche der Kornea und den gewählten Querschnittsverlauf des Schnitts auf dieser Strecke bestimmt. Der Schnitt eröffnet einen schlitzartigen Zugang zum Augeninneren durch die Kornea hindurch, wobei dieser Schlitz bei Betrachtung in Draufsicht auf die Vorderfläche der Kornea im wesentlichen geradlinig sein kann oder auch mehr oder weniger stark gekrümmt. Die in einer solchen Draufsicht gesehene Länge des Schlitzes (entsprechend der Breite des Schnitts) beträgt beispielsweise nur einige Millimeter. Sie kann je nach den Anforderungen des konkreten Anwendungsfalls festgelegt werden, insbesondere abhängig von der Größe der einzuführenden bzw. zu entnehmenden Instrumente und Materialien. Sofern mehrere Durchgangsschnitte in der Kornea präpariert werden, so kann zumindest eine Teilanzahl dieser Schnitte einen voneinander abweichenden Querschnittsverlauf von Vorderfläche zu Rückfläche der Kornea oder/und eine voneinander abweichende Breite aufweisen. Es versteht sich, dass zumindest eine Teilanzahl der Schnitte ebenso einen gleichen Querschnittsverlauf sowie eine gleiche Breite aufweisen kann.

Für eine gute Selbstdichtung der durch den Schnitt geöffneten Kornea empfiehlt es sich, wenn der Querschnittsverlauf des Schnitts mindestens einen Knick aufweist. Dabei kann der Querschnittsverlauf des Schnitts beidseits des Knicks je einen im wesentlichen geradlinigen Abschnitt aufweisen. Alternativ oder zusätzlich zu einer Einfach- oder Mehrfachknickung kann der Längsquerschnitt des Schnitts einen oder mehrere bogen- oder wellenförmige Segmente aufweisen. Auch so kann eine gute Selbstdichtung der Kornea erreicht werden.

Für eine besonders gute Selbstdichtung kann der Querschnittsverlauf des Schnitts mehrere Knicke aufweisen. Hierbei hat es sich als vorteilhaft erwiesen, wenn der Querschnittsverlauf des Schnitts mindestens einen zwischen zwei Knicken verlaufenden geradlinigen Abschnitt aufweist, welcher im wesentlichen senkrecht zur Augenoberfläche verläuft. Nachdem die Augenoberfläche des humanen Auges gekrümmt ist und die Senkrechte deswegen an verschiedenen Stellen der Augenoberfläche unterschiedlich orientiert ist, ist stets - wenn von einer bestimmten Orientierung des Querschnittsverlaufs oder eines Teils desselben zur Senkrechten der Augenoberfläche die Rede ist - eine Senkrechte an der Stelle des betreffenden Schnitts gemeint.

Sofern der Querschnittsverlauf des Schnitts mindestens drei geradlinige Abschnitte aufweist, wird eine Aufeinanderfolge dieser Abschnitte nach Art eines Zickzackmusters empfohlen.

Hinsichtlich der Lage des Schnitts im Auge kann sich dieser längs oder quer zu einer gedachten, zur Pupille des Auges im wesentlichen konzentrischen Kreislinie erstrecken, d.h. seine Breite erstreckt sich in dieser Richtung. Der Schnitt kann dabei beispielsweise tangential zu der Kreislinie verlaufen, er kann aber auch unter einem beliebigen Winkel zu der Kreislinie verlaufen. Die gedachte Kreislinie kann innerhalb oder außerhalb des Pupillenrands liegen (bei Betrachtung in einer Draufsicht auf das Auge entlang der Pupillenachse), sie liegt aber jedenfalls innerhalb des Limbus des Auges. Bei Betrachtung in einem Querschnitt in Breitenrichtung ist der Schnittverlauf zweckmäßigerweise geradlinig.

Der Schnitt kann auf seiner gesamten Länge von der Vorderfläche zur Rückfläche der Kornea im wesentlichen konstante Breite besitzen. Alternativ kann er eine sich zur Rückfläche hin verjüngende Breite besitzen, d.h. sich zur Rückfläche hin verjüngen (z.B. stetig oder stufenartig). Selbstverständlich ist es auch möglich, dass der Schnitt eine sich zur Rückfläche hin vergrößernde Breite besitzt.

Soweit Bedarf für die Präparation einer Mehrzahl Schnitte besteht, so sind die Instruktionen des Steuerprogramms vorzugsweise dazu ausgelegt, die Erzeugung jeweils zumindest eines Teils von mehreren Schnitten zu bewirken. Von diesen kann zumindest eine Teilanzahl von wenigstens zwei Schnitten längs einer gedachten, zur Pupille des Auges im wesentlichen konzentrischen Kreislinie verteilt angeordnet sein. Generell können die Schnitte auf mehrere gedachte, zur Pupille des Auges im wesentlichen konzentrische Kreislinien verteilt angeordnet sein. Die Zentrierung der erwähnten Kreislinien gegenüber dem Pupillenmittelpunkt bezieht sich hier auf eine Sichtweise in Draufsicht auf das Auge entlang der Pupillenachse des Auges, wobei es sich versteht, dass die hier erwähnten Kreislinien in einer Abwandlung auch exzentrisch zum Pupillenmittelpunkt liegen können.

In einer Ausgestaltung kann der mindestens eine Schnitt vollständig mit der Lasereinrichtung erzeugt werden, d.h. die Instruktionen des Steuerprogramms sind bei dieser Ausgestaltung dazu ausgelegt, bei Ausführung durch den Steuerrechner den Schnitt auf seiner gesamten von der Rückfläche bis zur Vorderfläche der Kornea durchgehenden Länge zu erzeugen. In einer alternativen Ausgestaltung kann die Lasereinrichtung so programmiert sein, dass sie nur einen von der Rückfläche der Kornea ausgehenden, jedoch im Abstand vor der Korneavorderfläche endenden Teil des Schnitts erzeugt. Beispielsweise endet dieser lasertechnisch erzeugte Teil des Schnitts höchstens etwa 100 µm, besser höchstens etwa 70 µm, unterhalb der Korneavorderfläche. Beispielsweise endet er etwa 50 µm unterhalb der Korneavorderfläche, d.h. etwa dort, bis wohin sich das korneale Epithel erstreckt. Es ist selbstverständlich möglich, dass der lasertechnisch erzeugte Teil des Schnitts in noch kürzerem Abstand von der Korneavorderfläche endet, etwa nur 20 oder 30 µm. In jedem Fall ist der Restteil der Kornea vorzugsweise dünner als derjenige Teil der Kornea, der von dem lasertechnisch erzeugten Teil des Schnitts durchsetzt ist. Dieser Restteil kann im Anschluss an die lasertechnische Schnitterzeugung vom Operateur mit einem herkömmlichen mechanischen Schneidinstrument (Skalpell) durchtrennt werden. Dies ermöglicht eine Behandlungsweise, bei welcher der lasertechnisch zu erzeugende Teil des Schnitts zunächst in einer unsterilen Umgebung erzeugt wird, ohne dass dies besondere Gefahren für den Patienten bergen würde, weil das Auge in dieser unsterilen Umgebung noch nicht vollständig eröffnet wird. Die Vervollständigung des Schnitts durch händische Durchtrennung des verbleibenden Reststücks der Kornea kann dann vom Operateur in einer sterilen Umgebung vorgenommen werden, in der auch die eigentliche Operation durchgeführt wird, für welche der Zugangskanal im Auge benötigt wird. Für eine solche Ausgestaltung kann die erfindungsgemäße Einrichtung zusätzlich zur Lasereinrichtung noch mindestens ein Skalpell umfassen, mit welchem der Operateur den Schnitt bis zur Korneavorderfläche vervollständigen kann.

Die Erfindung wird nachfolgend anhand der beigefügten Zeichnungen weiter erläutert. Es stellen dar:
- Fig. 1: in schematischer Blockdarstellung ein Ausführungsbeispiel einer Lasereinrichtung zur Anbringung von durchgehenden Inzisionen in der humanen Kornea,
- Fig. 2: schematisch ein beispielhaftes Anordnungsbild für mehrere durchgehende korneale Inzisionen,
- Fig. 3: schematisch eine beispielhafte korneale Inzision bei Betrachtung in einem x-z-Querschnitt durch die Kornea, und
- Fig. 4: die Inzision der Fig. 3 bei Betrachtung in einem y-z-Querschnitt durch die Kornea.

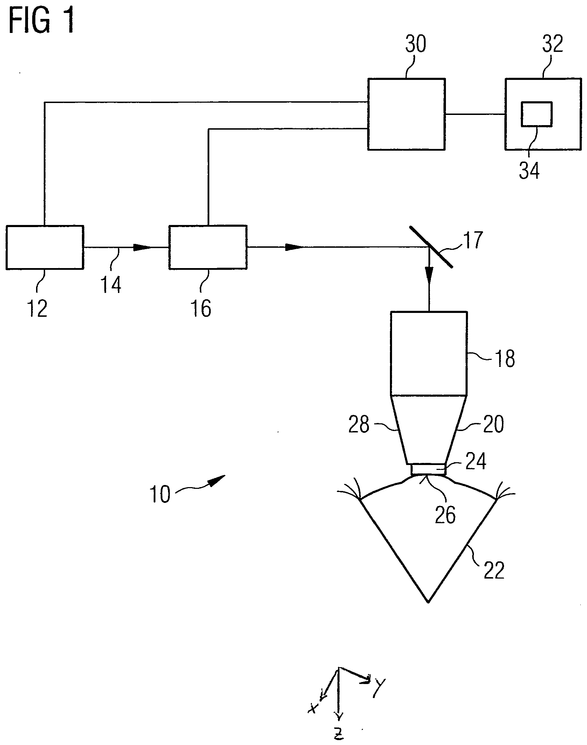

Es wird zunächst auf Fig. 1 verwiesen. Die dortige, allgemein mit 10 bezeichnete Lasereinrichtung umfasst eine Laserquelle 12, welche einen Laserstrahl 14 mit Pulsdauern im Femtosekundenbereich erzeugt. Im Strahlweg des Laserstrahls 14 sind eine Reihe von Komponenten angeordnet, unter anderem ein hier schematisch als einheitlicher Funktionsblock angedeuteter Scanner 16, ein unbeweglicher Umlenkspiegel 17 sowie ein Fokussierobjektiv 18. Der Scanner 16 dient zur transversalen und longitudinalen Ortssteuerung des Fokuspunkts des Laserstrahls 14. Transversal bezeichnet hier eine Richtung quer zur Ausbreitungsrichtung des Laserstrahls 14 im Bereich des Auges, longitudinal entspricht der Strahlsausbreitungsrichtung. In üblicher Notation wird die Transversalebene als x-y-Ebene bezeichnet, während die Longitudinalrichtung als z-Richtung bezeichnet wird. Eine entsprechendes x-y-z-Koordinatenkreuz ist in Fig. 1 zu Zwecken der Veranschaulichung eingezeichnet.

Zur transversalen Ablenkung des Laserstrahls 14 (d.h. in der x-y-Ebene) kann der Scanner 16 beispielsweise ein paar galvanometrisch betätigter Scannerspiegel umfassen, die um zueinander senkrechte Achsen kippbar angeordnet sind. Alternativ ist beispielsweise eine transversale Ablenkung mittels eines elektrooptischen Kristalls denkbar. Für die z-Steuerung der Fokusposition kann der Scanner 16 beispielsweise eine longitudinal verstellbare oder brechkraftvariable Linse oder einen deformierbaren Spiegel enthalten, mit der bzw. mit dem sich die Divergenz des Laserstrahls 14 und folglich die z-Position des Strahlfokus bei unveränderter Fokussiereinstellung des Fokussierobjektivs 18 beeinfilussen lässt.

Es versteht sich, dass die für die transversale Fokussteuerung und die longitudinale Fokussteuerung dienenden Komponenten des Scanners 16 entlang des Strahlwegs der Laserstrahls 14 verteilt angeordnet und insbesondere auf unterschiedliche Baueinheiten aufgeteilt sein können. Beispielsweise kann die Funktion der z-Fokusjustierung von einer in einer Strahlaufweitungsoptik (Beam Expander, z. B. Galileo-Teleskop) angeordneten Linse ausgefüllt werden, während die für die transversale Fokussteuerung dienenden optischen Komponenten in einer gesonderten Baueinheit zwischen der Strahlaufweitungsoptik und dem Fokussierobjektiv 18 untergebracht sein können. Die Darstellung des Scanners 16 als einheitlicher Funktionsblock in Fig. 1 dient lediglich der besseren Übersichtlichkeit.

Das Fokussierobjektiv 18 ist bevorzugt ein F-Theta-Objektiv und ist auf seiner Strahlaustrittsseite mit einem Patientenadapter 20 vorzugsweise lösbar gekoppelt, welcher eine Anlageschnittstelle für die Kornea eines zu behandelnden Auges 22 bildet. Der Patientenadapter 20 weist hierzu ein für die Laserstrahlung transparentes Kontaktelement 24 auf, welches auf seiner augenzugewandten Unterseite eine Anlagefläche 26 für die Kornea bildet. Die Anlagefläche 26 ist im gezeigten Beispielfall als Planfläche ausgeführt und dient zur Einebnung der Kornea, indem das Kontaktelement 24 mit entsprechendem Druck gegen das Auge 22 gedrückt wird oder die Kornea durch Unterdruck an die Anlagefläche 26 angesaugt wird.

Das Kontaktelement 24 (bei planparalleler Ausführung üblicherweise als Applanationsplatte bezeichnet) ist am schmäleren Ende einer sich konisch aufweitenden Trägerhülse 28 angebracht. Die Verbindung zwischen dem Kontaktelement 24 und der Trägerhülse 28 kann unlösbar sein, beispielsweise durch Verklebung, oder sie kann lösbar sein, etwa durch eine Verschraubung. Die Trägerhülse 28 besitzt in nicht näher dargestellter Weise an ihrem breiteren Hülsenende geeignete Koppelformationen zur Ankopplung an das Fokussierobjektiv 18.

Die Laserquelle 12 und der Scanner 16 werden von einem Steuerrechner 30 gesteuert, der nach Maßgabe eines in einem Speicher 32 gespeicherten Steuerprogramms 34 arbeitet. Das Steuerprogramm 34 enthält Instruktionen (Programmcode), welche bei Ausführung durch den Steuerrechner 30 eine solche Ortssteuerung des Strahlfokus des Laserstrahls 14 bewirken, dass in der Kornea des an dem Kontaktelement 24 anliegenden Auges 22 eine oder mehrere durchgehende, schlitzartige Inzisionen erzeugt werden.

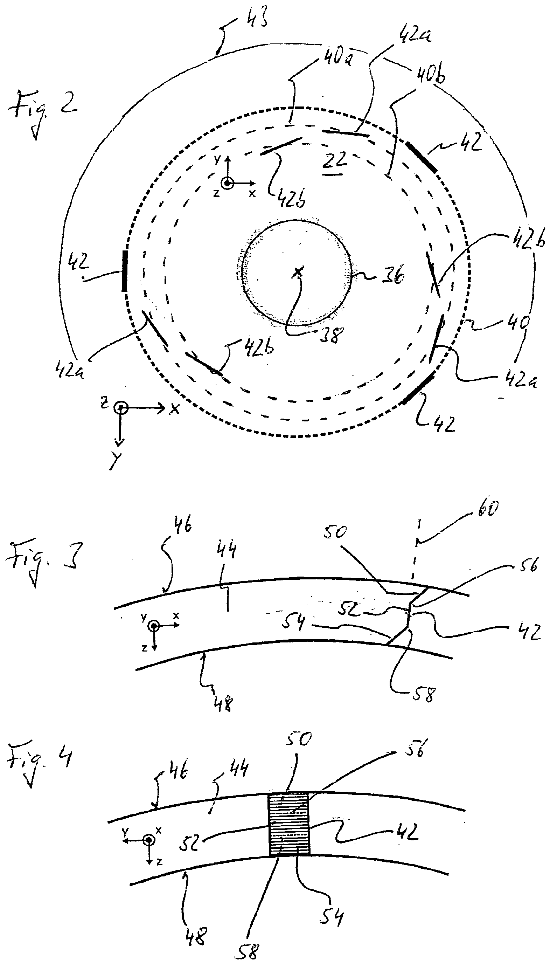

Ein mögliches Anordnungsbild dieser Inzisionen ist schematisch in Fig. 2 gezeigt. Eine Pupille 36 ist dort durch ihren Pupillenrand angedeutet. Die Pupille 36 besitzt ein Pupillenzentrum 38. Konzentrisch zum Pupillenzentrum 38 ist gestrichelt eine gedachte Kreislinie 40 eingezeichnet, welche die Pupille 36 in radialem Abstand außen umgibt. Längs dieser Kreislinie 40 in im wesentlichen gleichen Abständen verteilt sind drei Schlitzinzisionen 42 eingezeichnet, welche die Kornea des Auges 22 auf deren gesamter Dicke durchdringen und jeweils einen Zugangskanal zur Augenvorderkammer und zu den übrigen Innenbereichen des Auges eröffnen. Man erkennt, dass die Inzisionen 42 im Beispielfall der Fig. 2 näherungsweise gleiche Breite besitzen und in Richtung ihrer Breite geradlinig ausgeführt sind. Dabei liegen sie annährend tangential zur Kreislinie 40. Es versteht sich, dass mindestens eine Teilanzahl der Inzisionen 42 alternativ quer (unter einem beliebigen Winkel) zur Kreislinie 40 orientiert sein kann.

Es versteht sich ferner, dass die Anzahl der Inzisionen 42 variabel sein kann. Je nach durchzuführender Operation kann eine einzelne Inzision 42 genügen oder es können mehrere Inzisionen 42 nötig sein. Es ist auch nicht erforderlich, sämtliche Inzisionen 42 längs derselben Kreislinie 40 verteilt anzuordnen. Vorstellbar ist es, zumindest eine Teilanzahl der Inzisionen in unterschiedlichem radialen Abstand vom Pupillenzentrum 38 anzubringen. Dies kann beispielsweise durch eine exzentrische Lage der Kreislinie 40 gegenüber dem Pupillenzentrum 38 erreicht werden. Es kann alternativ erreicht werden, indem die Inzisionen auf mehrere zentrisch angeordnete Kreislinien verteilt werden, wie in Fig. 2 beispielhaft anhand der gestrichelt eingezeichneten zusätzlichen Kreislinien 40a, 40b und der weiteren Inzisionen 42a, 42b veranschaulicht. Mit 43 ist in dieser Figur der Limbus des Auges schematisch skizziert. Im Übrigen ist es auch nicht erforderlich, die Inzisionen längs einer Kreisbahn zu verteilen. Insgesamt besteht bezüglich der Verteilung in radialer Richtung sowie bezüglich der Verteilung in Umfangsrichtung keine prinzipielle Beschränkung für das Anordnungsbild der Inzisionen 42.

Die Inzisionen 42 können gleich oder unterschiedlich ausgestaltet sein. Für eine mögliche Ausgestaltung einer der Inzisionen wird nun auf die Figuren 3 und 4 verwiesen. Diesen zeigen transversale Ansichten derselben Inzision aus unterschiedlichen Blickrichtungen, wie durch die eingezeichneten xyz-Koordinatenkreuze veranschaulicht.

Die Kornea des zu bearbeitenden Auges ist in den Figuren 3 und 4 mit 44 bezeichnet. Sie besitzt eine Vorderfläche 46 sowie eine Rückfläche 48. In beiden Figuren ist sie in einem entspannten, nicht applanierten Zustand gezeigt (d. h. nach Wegnahme von dem Kontaktelement 24).

Die in den Figuren 3 und 4 gezeigte beispielhafte Inzision 42 zeigt bei einer transversalen Ansicht von der schmalen Schlitzseite her ein von der Vorderfläche 46 zur Rückfläche 48 verlaufendes Zickzack-Muster mit mehreren (hier drei) geradlinigen Abschnitten 50, 52, 54, die jeweils paarweise durch einen Knick 56, 58 getrennt sind. Der mittlere Abschnitt 52 verläuft im wesentlichen parallel zu einer gestrichelt eingezeichneten Normalen 60 zur Augenoberfläche (die Augenoberfläche ist hier gleichbedeutend mit der Vorderfläche 46 der Kornea). Es versteht sich, dass diese Betrachtung nur für eine Flächennormale im Bereich der Inzision 42 gilt, da in anderen Bereichen der Augenvorderfläche die jeweilige Flächennormale räumlich anders als die eingezeichnete Normale 60 orientiert ist.

Anstatt des in Fig. 3 gezeigten Zick-Zack- oder Sägezahnmusters ist es ohne weiteres vorstellbar, die Inzision 42 mit einem Wellenprofil auszuführen. Die Knicke 56, 58, sind dann durch rundliche Bögen ersetzt.

Die transversale Ansicht der Inzision 42 von der breiten Schlitzseite gemäß Fig. 4 verdeutlicht zudem, dass die Inzision 42 im gezeigten Beispielfall über ihre gesamte Länge im wesentlichen gleichbleibende Breite besitzt (wobei hier alternativ eine Verjüngung zur Rückfläche 48 hin vorstellbar ist). Für die Erzeugung der Inzision 42 wird der Fokus des verwendeten Laserstrahls in einem Linienraster in aufeinanderfolgenden Scanlinien bewegt, wobei zur Vermeidung möglicher Abschattungseffekte zweckmäßigerweise an der Rückfläche 48 der Kornea 44 mit der Schnitterzeugung begonnen wird. Von dort schreiten die einzelnen Scanlinien zunehmend in Richtung zur Vorderfläche 46 voran. Die in Fig. 4 fett eingezeichneten, in Schlitzbreite verlaufenden Striche verdeutlichen das Linienraster für den Strahlfokus bei der Erzeugung der Inzision 42.

## Patentansprüche

1. Einrichtung zur Erzeugung mindestens eines von der Rückfläche (48) bis zur Vorderfläche (46) der Kornea (44) eines humanen Auges durchgehenden schlitzförmigen, einen Zugangskanal zu innenliegenden Bereichen des Auges eröffnenden Schnitts (42), wobei die Einrichtung eine Lasereinrichtung zur Erzeugung wenigstens eines Teils des Schnitts mit fokussierter gepulster Laserstrahlung umfasst, wobei die Lasereinrichtung steuerbare Komponenten (16) zur Ortseinstellung des Strahlungsfokus, einen Steuerrechner (30) zur Steuerung dieser Komponenten und ein Steuerprogramm (34) für den Steuerrechner umfasst, wobei das Steuerprogramm Instruktionen enthält, welche dazu ausgelegt sind, bei Ausführung durch den Steuerrechner die Erzeugung zumindest eines von der Rückfläche (48) der Kornea ausgehenden Teils des Schnitts (42) zu bewirken, wobei zumindest dieser Teil des Schnitts einen Querschnittsverlauf - bei Betrachtung in Richtung von der Vorderfläche zur Rückfläche - aufweist, welcher von einer zur Augenoberfläche senkrechten Geraden (60) abweicht,
**dadurch gekennzeichnet, dass** der Querschnittsverlauf des Schnitts (42) mehrere Knicke (56, 58) aufweist und mindestens einen zwischen zwei Knicken (56, 58) verlaufenden geradlinigen Abschnitt (52) aufweist, welcher im Wesentlichen senkrecht zur Augenoberfläche verläuft.

2. Einrichtung nach Anspruch 1, wobei der Querschnittsverlauf des Schnitts (42) beidseits eines der Knicke (56, 58) je einen im wesentlichen geradlinigen Abschnitt (50, 52, 54) aufweist.

3. Einrichtung nach Anspruch 1 oder 2, wobei der Querschnittsverlauf des Schnitts (42) mindestens drei geradlinige Abschnitte (50, 52, 54) aufweist, welche nach Art eines Zickzackmusters aufeinanderfolgen.

4. Einrichtung nach einem der vorhergehenden Ansprüche, wobei sich der Schnitt (42) längs oder quer zu einer gedachten, zur Pupille (36) des Auges im wesentlichen konzentrischen Kreislinie (40) erstreckt.

5. Einrichtung nach einem der vorhergehenden Ansprüche, wobei der Schnitt (42) auf seiner gesamten Länge von der Vorderfläche (46) zur Rückfläche (48) der Kornea (44) im wesentlichen konstante Breite besitzt oder eine sich zur Rückfläche hin verringernde oder vergrößernde Breite besitzt.

6. Einrichtung nach einem der vorhergehenden Ansprüche, wobei die Instruktionen des Steuerprogramms dazu ausgelegt sind, die Erzeugung jeweils zumindest eines Teils von mehreren Schnitten (42) zu bewirken.

7. Einrichtung nach Anspruch 6, wobei zumindest eine Teilanzahl von wenigstens zweien der Schnitte längs einer gedachten, zur Pupille (36) des Auges im wesentlichen konzentrischen Kreislinie (40) verteilt angeordnet sind.

8. Einrichtung nach Anspruch 6 oder 7, wobei die Schnitte (42) auf mehrere gedachte, zur Pupille (36) des Auges im wesentlichen konzentrische Kreislinien verteilt angeordnet sind.

9. Einrichtung nach einem der vorhergehenden Ansprüche, wobei die Laserstrahlung Pulsdauern im Bereich von weniger als 1 Pikosekunde besitzt.

## Claims

1. Device for generating at least one slit-shaped incision (42) which extends from the posterior surface (48) as far as the anterior surface (46) of the cornea (44) of a human eye and opens up an access channel to internal areas of the eye, the device comprising a laser device for generating at least a part of the incision with focused pulsed laser radiation, the laser device comprising controllable components (16) for setting the location of the radiation focus, a control computer (30) for controlling these components, and a control program (34) for the control computer, the control program containing instructions which, when executed by the control computer, are designed to bring about the generation of at least a part of the incision (42) originating from the posterior surface (48) of the cornea, at least this part of the incision having a cross-sectional profile which, viewed in the direction from the anterior surface to the posterior surface, deviates from a straight line (60) perpendicular to the surface of the eye, **characterized in that** the cross-sectional profile of the incision (42) has a plurality of kinks (56, 58), and it has at least one rectilinear portion (52), which extends between two kinks (56, 58) and extends substantially perpendicularly with respect to the surface of the eye.

2. Device according to Claim 1, wherein the cross-sectional profile of the incision (42) has a substantially rectilinear portion (50, 52, 54) on both sides of each of the kinks (56, 58).

3. Device according to Claim 1 or 2, wherein the cross-sectional profile of the incision (42) has at least three rectilinear portions (50, 52, 54), which follow one another in the manner of a zigzag pattern.

4. Device according to one of the preceding claims, wherein the incision (42) extends along or across an imaginary circular line (40) which is substantially concentric to the pupil (36) of the eye.

5. Device according to one of the preceding claims, wherein the incision (42) has a substantially constant width over its entire length from the anterior surface (46) to the posterior surface (48) of the cornea (44) or has a width that decreases or increases towards the posterior surface.

6. Device according to one of the preceding claims, wherein the instructions of the control program are designed to bring about the generation in each case of at least some of a plurality of incisions (42).

7. Device according to Claim 6, wherein at least a partial number of at least two of the incisions are arranged distributed along an imaginary circular line (40) which is substantially concentric to the pupil (36) of the eye.

8. Device according to Claim 6 or 7, wherein the incisions (42) are arranged distributed on several imaginary circular lines which are substantially concentric to the pupil (36) of the eye.

9. Device according to one of the preceding claims, wherein the laser radiation has pulse durations in the range of less than 1 picosecond.

## Revendications

1. Dispositif pour produire au moins une incision (42) en forme de fente, traversant la surface arrière (48) de la cornée (44) d'un oeil humain jusqu'à la surface avant (46), ouvrant un conduit d'accès aux zones situées à l'intérieur de l'oeil, le dispositif comprenant un dispositif laser pour produire au moins une partie de l'incision avec un faisceau laser pulsé focalisé, le dispositif laser comprenant des composants commandables (16) pour l'ajustement du site de focalisation du faisceau, un ordinateur de commande (30) pour commander ces composants et un programme de commande (34) pour l'ordinateur de commande, le programme de commande contenant des instructions qui sont conçues, dans le cas de l'exécution par l'ordinateur de commande, pour provoquer la génération d'au moins une partie de l'incision (42) partant de la surface arrière (48) de la cornée, au moins cette partie de l'incision présentant une allure en section transversale - vu dans la direction allant de la surface avant vers la surface arrière - qui s'écarte d'une ligne droite (60) perpendiculaire à la surface de l'oeil,
**caractérisé en ce que** l'allure en section transversale de l'incision (42) présente plusieurs points d'inflexion (56, 58) et au moins une portion rectiligne (52) s'étendant entre deux points d'inflexion (56, 58), qui s'étend essentiellement perpendiculairement à la surface de l'oeil.

2. Dispositif selon la revendication 1, dans lequel l'allure en section transversale de l'incision (42) présente, de part et d'autre de l'un des points d'inflexion (56, 58), à chaque fois une portion essentiellement rectiligne (50, 52, 54).

3. Dispositif selon la revendication 1 ou 2, dans lequel l'allure en section transversale de l'incision (42) présente au moins trois portions rectilignes (50, 52, 54) qui se suivent à la manière d'un motif en zigzag.

4. Dispositif selon l'une quelconque des revendications précédentes, dans lequel l'incision (42) s'étend le long de ou transversalement à une ligne circulaire imaginaire (40) essentiellement concentrique à la pupille (36) de l'oeil.

5. Dispositif selon l'une quelconque des revendications précédentes, dans lequel l'incision (42) possède, sur toute sa longueur depuis la surface avant (46) jusqu'à la surface arrière (48) de la cornée (44), essentiellement une largeur constante ou une largeur diminuant ou augmentant vers la surface arrière.

6. Dispositif selon l'une quelconque des revendications précédentes, dans lequel les instructions du programme de commande sont conçues pour provoquer la génération à chaque fois d'au moins une partie de plusieurs incisions (42).

7. Dispositif selon la revendication 6, dans lequel des incisions, au moins en un nombre partiel d'au moins deux, sont disposées de manière répartie le long d'une ligne circulaire imaginaire (40) essentiellement concentrique à la pupille (36) de l'oeil.

8. Dispositif selon la revendication 6 ou 7, dans lequel les incisions (42) sont disposées de manière répartie sur plusieurs lignes circulaires imaginaires, essentiellement concentriques à la pupille (36) de l'oeil.

9. Dispositif selon l'une quelconque des revendications précédentes, dans lequel le faisceau laser possède des durées d'impulsions de l'ordre de moins de 1 picoseconde.
